(19) Europäisches Patentamt
European Patent Office
Office européen des brevets

(11) Publication number: **0 200 213 B1**

(12) **EUROPEAN PATENT SPECIFICATION**

(45) Date of publication of patent specification: **22.07.92**

(51) Int. Cl.$^5$: **A61K 47/00**, A61K 9/16, A61K 9/22

(21) Application number: **86105925.1**

(22) Date of filing: **29.04.86**

# (54) Hydrogels with increased organic solvent soluble active agent loading capacity, their preparation and the use thereof.

(30) Priority: **02.05.85 US 729821**

(43) Date of publication of application:
**05.11.86 Bulletin 86/45**

(45) Publication of the grant of the patent:
**22.07.92 Bulletin 92/30**

(84) Designated Contracting States:
**AT BE CH DE FR GB IT LI LU NL SE**

(56) References cited:
**EP-A- 0 140 828**
**FR-A- 2 391 721**
**US-A- 4 177 056**

(73) Proprietor: **CIBA-GEIGY AG**
**Klybeckstrasse 141**
**CH-4002 Basel(CH)**

(72) Inventor: **Good, William R.**
**18, Marybeth Drive**
**Suffern New York 10901(US)**
Inventor: **Mikes, John**
**16 Dixwell Road**
**New City New York(US)**
Inventor: **Sikora, Joseph**
**Box 71 Conklinton Town Road**
**Wanaque New Jersey 07456(US)**

(74) Representative: **Zumstein, Fritz, Dr. et al**
**Bräuhausstrasse 4**
**W-8000 München 2(DE)**

EP 0 200 213 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to improved water-insoluble hydrogels which are in the form of macromer-crosslinked polymers of one or more water-soluble monoolefinic monomers, optionally containing a minor amount of one or more water-insoluble monoolefinic monomers having superior active agent loading capacity. The macromer component is a terminally diolefinic hydrophobic macromer containing a polypropylene oxide or polytetramethylene oxide diradical in the macromer chain. These hydrogels can be loaded with active agents, especially biologically active agents, including pharmaceuticals, insecticides, herbicides, for the controlled, sustained release thereof by diffusion upon contact with an aqueous environment.

FR-A1 2,391,721 discloses copolyesters but does not mention the hydrogel components (A) or (B) as disclosed hereinafter. Components (A) and (B) as disclosed herein and hydrophilic hydrogels in the form of macromer-crosslinked polymers containing monomer and macromer components are known, for example, from US-A-4,177,056 disclosing eligible components, including active agents, monomers and macromers; polymer preparation, including process parameters and modes of preparation; active agent loading techniques, and the use thereof; and preferred embodiments in respect to such components, preparation, loading techniques and use. However, said hydrogels are not expanded.

JP-A-60-67435, being equivalent to EP-A-140,828, discloses copolymers which are expanded with single compounds comprised by formula II as defined hereinafter. However, the comonomers used for the manufacture of the copolymers according to EP-A-140,828 are predominantly water-insoluble (at least 55 % thereof are water-insoluble). This is in contrast to the invention where the comonomers are predominantly water-soluble (at least 50 % thereof are water-soluble). Surprisingly this difference translates into unexpected properties since the expanded hydrogels of the invention are substantially free from macroporosity, whereas according to JP-A-60-67435 hydrogels having macroporous structures are obtained.

Thus, it has been surprisingly and unexpectedly discovered that the nature of macromer-crosslinked polymers can be substantially and irrevocably modified so as to increase the organic solvent swelling characteristics of the polymers. As a result, the amount of organic solvent soluble active agent capable of being loaded into such polymers can be greatly increased.

This modification of the macromer-crosslinked polymers is obtained by conducting the polymerization in the presence of certain macromer compatible compounds. Such compounds act as macromer expanders by increasing the organic solvent soluble active agent loadability. But, by virtue of their solubility in the macromer component, both prior to and during polymerization, the macromer expanding compounds do not occasion the formation of copolymers having a macroporous structure. Macroporous copolymers are generally characterized in having pores ranging from about 10-500 nm (100-5000 Angstroms). Various crosslinked macroporous polymers are disclosed, for example, in US Patent No. 3,509,078. In contrast to macroporous copolymers, the instant macromer expanded copolymers are substantially free from macroporosity. As a result, the instant glossy, i.e., optically clear to hazy, copolymers release the active agent by controlled diffusion upon contact with an aqueous environment, in contrast to convectional paths, occasioned by macroporous networks.

It is an object of the present invention to provide novel macromer expanded hydrogel polymers having substantially increased organic solvent soluble active agent loadability and their preparation and use.

It is a further object of the present invention to provide active agent loaded controlled, sustained release compositions of such polymers and their preparation and use.

These and other objects of the present invention are apparent from the following description.

One embodiment of the present invention relates to a process for the production of a controlled, sustained release composition comprising:

(a) an organic-solvent soluble active agent in an amount sufficient for the total desired dosage during the release period and distributed within

(b) a water-insoluble macromer expanded hydrogel comprising the crosslinked copolymerization product of

(A) 30 to 90 % by weight of said hydrogel of (a') a water-soluble monoolefinic monomer, or mixture of said monomers, or (b') a water-soluble monoolefinic monomer, or mixture of said monomers with 1 to 50 % by weight of total monomers of a water-insoluble monoolefinic monomer, or mixture of said water-insoluble monomers, with

(B) 70 to 10 % by weight of said hydrogel of a terminal diolefinic hydrophobic macromer having a molecular weight from 400 to 8000, said macromer having the formula I,

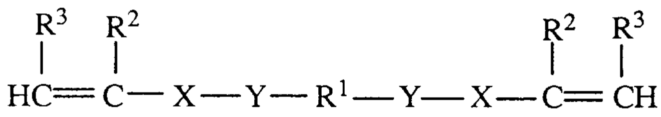 (I)

wherein $R^1$ is a polycondensate chain having a molecular weight from 200 to 4000, which is the residue of a poly (propylene oxide) or poly (tetramethylene oxide) glycol having ether linkages; $R^2$ is hydrogen, methyl or $-CH_2COOR^4$ wherein $R^4$ is hydrogen or an alkyl group with up to ten carbon atoms; $R^3$ is hydrogen or $-COOR^4$, with the proviso that at least one of $R^2$ and $R^3$ is hydrogen; X is oxygen, -COO-, or-$CONR^5$- wherein $R^5$ is hydrogen or alkyl of up to 5 carbon atoms and Y is a direct bond or the radical $-R^6-Z^1-CO-NH-R^7-NH-CO-Z^2-$, wherein $R^6$ is linked to X and represents branched or linear alkylene of up to 7 carbon atoms, $Z^1$ and $Z^2$ are oxygen or $NR^5$, and $R^7$ is the diradical of an aliphatic, including cycloaliphatic, or aromatic diisocyanate, with the proviso that in case X is oxygen, Y is different from a direct bond, and $R^2$ and $R^3$ are hydrogen; comprising the steps of copolymerizing said monomer(A) and said macromer (B) in the presence of a free radical initiator at a reaction temperature between 20 to 150 ° C, in the presence or absence of an inert diluent, to form said hydrogel, and loading said active agent (a) into said hydrogel, characterized in that the copolymerizing step is made in the presence of 5 to 70 weight percent of the total weight of (A) and (B) of a macromer (B) soluble compound of the formula II,

$$R'\!-\!\!\left(R''-O\right)_n\!-\!R''' \qquad \text{(II)}$$

wherein R' is hydroxy, alkoxy of up to eight carbon atoms or alkanoyloxy of up to eight carbon atoms; R'' is straight or branched chain alkylene of two to six carbon atoms; R''' is hydrogen, alkyl of up to eighteen carbon atoms or alkanoyl of up to eighteen carbon atoms; and n is an integer from 1 to 100, with the proviso that if n is 1 or R'' is ethylene, R''' is alkyl of two to eighteen carbon atoms or alkanoyl of three to eighteen carbon atoms, or a mixture thereof.

An alternate embodiment of the invention relates to the water-insoluble macromer expanded hydrogel itself, useful as an intermediate in preparation of the active agent loaded composition, and prepared in accordance with the preceeding paragraph, excluding the loading of active agent (a) into said hydrogel.

A further alternate embodiment relates to the active agent loaded composition produced, and its use in a method of releasing the active agent; such as the oral administration of an effective amount of a pharmaceutically effective active agent to a host, e.g. a patient, in need of such active agent.

The amount of compound of formula II or mixture thereof incorporated in the monomer/macromer mixture during, or preferably prior to, the polymerization step is between 5 to 70 weight percent, preferably between 10 and 60 weight percent, of the total weight of monomer (A) and macromer (B). The amount of increased organic solvent swellability, as measured using one or more conventional organic swelling solvents, is generally proportional to the amount of compound of formula II employed. Suitable organic swelling solvents for determining increased swellability, and for loading the macromer expanded hydrogels by dissolving the active agent (a) into said solvent and inbibing the active agent containing solution into the hydrogel, include lower alkanols, such as methanol or ethanol; lower alkanones, such as acetone or methyl ethyl ketone; di-lower alkyl ethers, such as ethyl ether; lower alkanoic acids, such as propionic acid; lower alkyl esters of lower alkanoic acids, such as ethyl acetate; lower alkylene oxides, such as tetrahydrofuran; and lower partially halogenated alkanes, such as dichloromethane or chloroform. Especially convenient are relatively volatile organic polar solvents, such as methanol, ethanol, dichloromethane or mixtures thereof. Water may be present in such organic solvents, and, in certain cases aqueous organic solvent systems, such as an aqueous ethanol solvent, have been found to be highly advantageous as a suitable organic solvent swelling medium, e.g. for loading active agents soluble therein.

The amount of increased organic solvent swellability of the instant macromer expanded hydrogels in comparison to their non-macromer expanded hydrogel counterparts is preferably between about 5 to about 120 %, most preferably between about 20 to about 90 %, by weight, using ethanol as a swelling solvent. Generally the macromer expander of formula II is removed prior to such comparative swelling.

While the organic solvent swellability, e.g. as measured using ethanol, can be increased dramatically using the instant macromer expanded hydrogels, thereby providing for an increased loadability of organic solvent soluble active agent, in many cases the aqueous swellability, using e.g. water as a swelling agent, is

increased only slightly or not at all. As a result, an additional advantage is realized in such cases, since the increase in loading capability of active agent can be achieved without significant increase in the rate of diffusion of active agent into the desired environment upon contact of the loaded composition with an aqueous medium.

Suitable compounds of formula II, as indicated above, must be soluble in the macromer component both prior to and during polymerization, such that the polymerization proceeds in essentially a single phase. Accordingly, those compounds of formula II, wherein n is 1, R' is hydroxy and R''' is hydrogen, such as ethylene glycol or propylene glycol, or where R' is hydroxy, R''' is hydrogen and R'' is ethylene, such as polyethylene oxide, are unsuitable due to their insufficient solubility, or compatibility, in the macromer component. However, derivatives of such compounds, wherein R''' is sufficiently hydrophobic, such as ethylene glycol monobutyl ether or ethylene glycol monobutyl ether acetate possess sufficient solubility or miscibility in the macromer component prior to and during polymerization so as to result in the desired macromer expanded polymer.

In a preferred embodiment of the compounds of formula II, R' is hydroxy or alkanoyloxy of up to three carbon atoms, R'' is alkylene of two to four carbon atoms, n is 1 and R''' is alkyl of three to six carbon atoms. Examples of such preferred compounds include, for example, ethylene glycol monobutyl ether, and ethylene glycol monobutyl ether acetate.

An alternate preferred embodiment of the compounds of formula II includes compounds of the formula III,

$HO\{R''-O\}_nH$ (III)

where n has an average value between about 8 and about 80 and R'' is alkylene of three or four carbon atoms. Highly preferred are compounds of formula III, wherein n has an average value between about 25 and 80 and R'' is isopropylene and also those compounds of formula III, wherein n has an average value between about 8 and 40 and R'' is tetramethylene. Suitable such compounds include polypropylene glycols having an average molecular weight of about 2000 or about 4000, and polytetramethylene glycols having an average molecular weight of about 650, 1000, and 2000.

Preferred monomers (A) include those water soluble monomers which are acrylic or methacrylic acid or water-soluble esters, amides or imides thereof, especially acrylic or methacrylic acid, or hydroxyalkyl or dialkylaminoalkyl esters thereof in which alkyl in each case has two to four carbon atoms. Suitable water-soluble monomers for use in accordance with the instant invention are recited in US Patent No. 4,177,056 and include, for example, acrylic acid, methacrylic acid, 2-hydroxyethyl or 2- or 3-hydroxypropyl acrylate or methacrylate, N-vinylpyrrolidone or tertiary methyl aminomethyl acrylamide, or mixtures thereof. Most preferred monomers are 2-hydroxyethyl methacrylate and N-vinylpyrrolidone.

Preferred water-insoluble olefinic monomers include alkyl acrylates or methacrylates, where alkyl has one to eighteen carbon atoms, e.g. methyl and ethyl methacrylate or acrylate; vinyl esters of alkanoic acids of up to five carbon atoms, e.g. vinyl acetate; acrylonitrile, styrene, and vinyl alkyl ethers where alkyl has up to five carbon atoms, e.g. ethyl vinyl ether.

Preferred macromer (B) components include those of formula I wherein the macromer is a reaction product of i) a polypropylene or polytetramethylene oxide glycol with a molecular weight of about 600 to about 4000, which is endcapped for example, with isophorone diisocyanate and ii) two moles, per mole of said glycol, of a hydroxyalkyl acrylate or methacrylate of 2 to 4 carbon atoms in the alkyl moiety.

The polymerization can be carried out in the presence of a conventional free radical initiator, including peroxides, such as benzoyl peroxide or t-butyl peroctoate; percarbonates, such as diisopropyl percarbonate; azonitriles, such as azoisobutyronitrile; persulfates, such as sodium persulfate; or other free radical generators, such as gamma rays, electron beams, and ultraviolet radiation.

The reaction temperature may vary widely and is limited only by the stability of the ingredients. Suitable reaction temperatures range from about 20°C to about 150°C, preferably between about 50°C to about 100°C. The hydrogel containing macromer may be prepared in the presence or absence of an inert diluent, in a closed mold, e.g. to form flat sheet or cylinder. Alternatively, the hydrogel may be prepared in the form of small spheres or beads, comprising the high speed stirring of the macromer, monomers, expander, catalyst and optionally active agent in a viscous medium which is not a solvent for any part of the hydrogel composition at a temperature between about 50°C and 100°C. Examples of suitable bead polymerization media are silicone oils, polyfluorinated oils, mineral spirit and saturated aqueous salt solutions.

Incorporation of the active agent into the hydrogel article may be accomplished either by dissolution or dispersion in the macromer solution prior to or during the copolymerization of macromer and monomer, or

by diffusion into the macromer expanded hydrogel from an organic solvent medium containing the active agent subsequent to the copolymerization. In this latter embodiment, the macromer expanded hydrogel may be washed with a solvent to remove the compound of formula II, prior to the incorporation, or loading, of the hydrogel with active agent. Suitable solvents for use in removing the compound of formula II from the macromer hydrogel include those organic swelling solvents recited above. After removal of the compound of formula II from the macromer hydrogel, the hydrogel may be dried, e.g. by solvent evaporation. Likewise, after the active agent is loaded into the hydrogel from a suitable solvent medium, the active agent loaded hydrogel can be dried by solvent evaporation. Upon contact with an aqueous environment, the active agent will be released in a controlled sustained manner.

Suitable active agents are pharmaceuticals, herbicides, insecticides, flavoring agents, nutrients, fungicides, bacteriocides, and the like, which are at least partially soluble in the chosen organic swelling solvent. Suitable active agents include, without limitation, those listed in US Patent No. 3,732,865 (Columns 10 and 11), US Patent No. 4,177,056 (Columns 10 and 11) and US Patent No. 3,660,563 (Columns 3 to 7). Preferred active agents include pharmaceuticals, herbicides, and insecticides. In the field of pharmaceuticals, the loaded hydrogels can be activated by ingestion into the gastrointestinal tract, wherein the active pharmaceutical is released upon contact with aqueous gastrointestinal fluid in a controlled sustained manner. For agricultural purposes, the active agent loaded hydrogel can be incorporated into the soil to release the active herbicide, insecticide, nutrient, or the like, in the presence of moist soil conditions.

The amount of active agent loaded into the macromer hydrogel will vary widely depending on the desired effect, the active agent employed, and the time span for which it takes the active agent to be released. For pharmaceutical applications, for example, the upper and lower limits of pharmaceutical agent incorporated will depend on the activity of the pharmaceutical, e.g. drug, and the span of its release in the carrier. Preferred drugs to be incorporated are those designed for long-term treatment so that multiple daily doses can be avoided, for example, anabolics; analgesics; anti-inflammatories, e.g. diclofenac sodium, aspirin, phenylbutazone or methadone; antibiotics, e.g. rifampin; antidepressants, e.g. imipramine or maprotiline; anticonvulsives, e.g. carbamazepine; antihypertensives, e.g. hydralazine; antiparasitics, e.g. nifurtimox; bronchodilators, e.g. fenoterol; coronary dilators, e.g. fenalcomine; corticoids, e.g. dexamethasone; diuretics, e.g. hydrochlorothiazide; hypnotics, e.g. glutethimide; neuroleptics, e.g. reserpine; tranquilizers, e.g. diazepam; or vasodilators, e.g. isoproterenol. Preferred pharmaceuticals include diclofenac sodium and carbamazepine.

The loaded macromer expanded hydrogels are advantageously stored in the dry state, e.g. by removal of the loading organic solvent from the loaded hydrogels by evaporation.

The following examples are set forth as illustrative of the instant invention. All parts are by weight unless otherwise specified. Trade names and registered trade marks are acknowledged as such.

Example 1: Bulk Polymerization of a Hydrogel

A glass plate is framed with 0.5 mm thick teflon rims and covered, with mylar foil, leaving a small opening at a corner of mold. Nitrogen is passed through for 10 minutes. A monomer/macromer is charged in through the opening and vacuum-treated to make it bubble-free.

The monomer/macromer is prepared by dissolving 600 g (0.24 moles) of a poly-(tetramethylene oxide) glycol with an average molecular weight of 2000 endcapped with isophorone diisocyanate in 900 g (7 moles) of 2-hydroxyethyl methacrylate (HEMA) and allowing said mixture to react for 72 hours at 30 °C. At the end of this period, the disappearance of the terminal isocyanate group is verified by noting the absence of the characteristic infrared spectral band at 2270 $cm^{-1}$ associated with the -NCO group. The composition of the monomer/macromer made of 900 g (60 %) HEMA (hydroxyethyl methacrylate) and 600 g (40 %) isocyanate endcapped polyether is marked as a "6040" composition. Similar nomenclature is used throughout the examples for the composition of the amphiphilic copolymers. 0.02 % of azo-bis-isobutyronitrile initiator is added to the monomer/macromer charged into the flat cavity between glass and mylar sheet bubble-free. After closing the corner-opening of the mold the assembly is placed in a 70 °C oven for 4 hours. Subsequently, the polymer film is removed from the mold; extracted free from residual monomers, oligomers, and other impurities and kept wet for further testing.

Example 2:

Same procedure is followed as in example 1, with the exception that 0.24 moles of poly-(propylene oxide) glycol (molecular weight of 2000) are used instead of the tetramethylene compound in the synthesis of the macromer.

Example 3:

Same procedure is followed as in example 1, with the exception of dissolving 25 g of P-PRG-2000, i.e. poly-(propylene oxide) glycol (molecular weight 2000), in 100 g of the monomer/macromer mixture before charging it into the mold. The polymer film is carefully extracted with alcohol until all expander is removed.

Example 4:

Same procedure is performed as described in example 2 using the variance as described in example 3.

Example 5:

The procedure as described in example 1 is followed with the variance of adding 40 g of EB-AC, i.e. ethylene glycol monobutyl ether acetate as macromer expander, to 100 g of the monomer/macromer before charging it into the mold. The polymer film is boiled in water under reduced pressure to remove the expander by an azeotropic distillation.

Example 6:

The process as described in example 2 is followed with the variance described in example 5.

Table I (Examples 1 to 6)

| Polymer of Example | Expander Type | Expander Percentage | Degree of Swelling (1) in 100 % water | Degree of Swelling (1) in 100 % ethanol |
|---|---|---|---|---|
| 1 | - | 0 | 20.7 | 56.8 |
| 2 | - | 0 | 22.1 | 83.0 |
| 3 | P-PRGL-2000 (*) | 25 | 19.1 | 66.5 |
| 4 | P-PRGL-2000 (*) | 25 | 21.9 | 101.0 |
| 5 | EB-AC | 40 | 17.6 | 79.8 |
| 6 | EB-AC | 40 | 22.3 | 121.0 |

(*) Polyglycol-2000, Dow.

(1) the degree of swelling (L, given in percent) is calculated by the formula

$$\frac{(S-D) \cdot 100}{D} = L ,$$

wherein S is the weight of swollen polymer and D is the weight of the dry polymer.

Example 7: Suspension Polymer Preparation - 6040

A smooth wall, 1000 ml resin flask is equipped with a reflux condenser, nitrogen inlet tube, thermometer - attached to a thermoregulator - and a paddle type stirrer driven by a variable motor. A slow nitrogen purge is maintained through the system at all times.

Charged into the flask are 360 g of a 20 % by weight aqueous sodium chloride solution containing 41 g (0.2 moles) of magnesium chloride hexahydrate. The solution is heated to 80 ° C and with a rapid stirring 123 ml (0.123 moles) of 1.0 N sodium hydroxide is added dropwise. When all the sodium hydroxide is added, the stirring speed is adjusted to 180 rpm and 180 g of monomer/macromer mix - with the initiator in it as described in example 1 - is added. After three hours the temperature is raised to 100 ° C for one more hour. The reaction is cooled to dissolve the magnesium hydroxide suspension agent. The isolated beads are washed and extracted in alcohol to remove any residual monomer or oligomer and other impurities. After eight hours, vacuum drying at 60 ° C, 173 g (95 % yield) bead polymer are obtained with an average diameter of 0.9 mm.

Example 8: Suspension Polymer Preparation - 6535

The procedure of example 7 is followed with only one variance: The composition of the monomer/macromer as described in example 1 is changed by dissolving the 600 g isocyanate-endcapped poly-(butylene oxide) glycol in 1114 g HEMA (weight ratio 35 to 65). The rest of the procedure is identical to the one described in the preceding example. Yield is 173.5 g (96.4 %), with an average bead diameter of 0.85 mm.

Example 9 and 10:

The process of example 7 is applied with the exception of using mixtures of monomer/macromer and butylene glycol mono-ethyl ether acetate (Ektasolve EBAC - Eastman Kodak) as expander/additive. In preparation of the 9 mixture, the 180 g monomer/macromer phase consists of 36 g EBAC and 144 g monomer/macromer; in 10 the mix contains 112.5 g monomer/macromer and 67.5 g EBAC. The post-heat of the process is carried out at 92 kPa pressure with a descending condenser attached to the flask and continued with constant replacement of the distillate volume by deonized water until the condensate is free of the EBAC odor - about 2 hours. Yield: 136 g (94.4 %) for example 9 product and 105.3 g (93.6 %) for the polymer of example 10, average bead diameters: 0.72 and 0.61 mm, respectively.

Example 11 and 12:

Using the procedure described in example 8, the process of copolymerization is performed as shown in the preceding examples 9 and 10. The composition of the monomer/macromer phases containes in example 11: 144 g monomer/macromer and 36 g Ektasolve EB (ethylene glycol monobutyl ether, Eastman Kodak) and in example 12 to 120 g monomer/macromer 60 g of the EB additive. The yields are 136.8 g (95 %) with an average bead diameter of 0.72 mm and 113.4 g (94.5 %), average diameter 0.64 mm, respectively.

Table II (Examples 7 to 12)

The products of examples 7 to 12 are tested for degree of swelling in water and ethanol and some are tested for the loading capacity and release rate of diclofenac sodium as an active ingredient.

| Polymer Exam-ple | Compo-sition | Expander Type and Percentage added | | Degree of Swelling [%] (1) | (2) | Loading Capacity [%] | Release Parameters T-50 (3) | T-90 (4) |
|---|---|---|---|---|---|---|---|---|
| 7 | 6040 | - | 0 | 27 | 60 | 28.2 | 6.95 | 31 |
| 8 | 6535 | - | 0 | 32 | 64 | 30.0 | 2.33 | 21.3 |
| 9 | 6040 | EB-AC | 25 | 32 | 87 | * | | |
| 10 | 6040 | EB-AC | 60 | 41 | 122 | 46 | 1.41 | 21 |
| 11 | 6535 | EB | 25 | 38 | 95 | * | | |
| 12 | 6535 | EB | 50 | 43 | 115 | * | | |

(1) Swelling in 100 % water

(2) Swelling in 100 % ethanol

(3) Hours elapsed while releasing 50 % active ingredient

(4) Hours elapsed while releasing 90 % active ingredient

* not applicable

Example 13: Suspension Polymer Preparation - 5050

Using the procedure of example 7 with 180 g monomer/macromer mix of 600 g HEMA and 600 g of the isocyanate endcapped poly-(butylene oxide) glycol, molecular weight 2000 (polymeg 2000 of Quaker Oats), the yield is 176.5 g (98.9 % bead copolymer of an average diameter of 1.1 mm).

Example 14 to 18:

Expanded copolymer hydrogels using the procedure of example 13 with poly-(propylene oxide) glycol, molecular weight 2000 (polyglycol 2000 of Dow) as the expanding additive, the compositions of the phases, yields and average particle size values, measured after an alcoholic extraction of the additive from the beads; are

| Example | monomer/macromer | PG 2000 | Yield | Average Particle Size |
|---|---|---|---|---|
| 14 | 171.4 g | 8.6 g | 167.6 g (98 %) | 1.1 mm |
| 15 | 163.3 g | 17.8 g | 157.3 g (97.2 %) | 1.0 mm |
| 16 | 156.5 g | 23.5 g | 150.0 g (98 %) | 1.0 mm |
| 17 | 150.0 g | 30.0 g | 142.5 g (99 %) | 0.98 mm |
| 18 | 144.0 g | 36.0 g | 131.0 g (97 %) | 0.99 mm |

Swelling tests are performed and diclofenac loadability % is assayed. The results are as follows:

Table III

| (Examples 13 to 18) | | | | | | |
|---|---|---|---|---|---|---|
| Polymer | | Expander | | Degree of Swelling [%] | | Loading Capacity [%] |
| Example | Composition | Type and Percentage added | | (1) | (2) | |
| 13 | 5050 | - | 0 | 21 | 54 | 26 |
| 14 | 5050 | PG 2000 | 5 | 23 | 56 | 27 |
| 15 | 5050 | PG 2000 | 10 | 23 | 57 | 29 |
| 16 | 5050 | PG 2000 | 15 | 20 | 62 | 29 |
| 17 | 5050 | PG 2000 | 20 | 22 | 67 | 32 |
| 18 | 5050 | PG 2000 | 25 | 20 | 69 | 32 |

(1) Swelling in 100 % water
(2) Swelling in 100 % ethanol

Example 19 to 25:

A similar series is prepared to the examples 13-18, using the procedure of example 8. With expander additive amounts of poly-(butylene oxide) glycol, molecular weight 1000 (polymeg 1000 of Quaker Oats), the compositions of the monomer/macromer phases in runs 19-23 are identical to the amounts given in examples 14-18, respectively. Example 24 containes 138.5 g monomer/macromer plus 41.5 g PM-1000 and example 25 is polymerized from a phase containing 133.33 g monomer/macromer with 46.66 g PM-1000. Yields and average particle sizes in the examples 19-25 are:

| | | |
|---|---|---|
| 19: 170 g | (99 %), | 0.84 mm; |
| 20: 158.9 g | (98.1 %), | 0.86 mm; |
| 21: 149.3 g | (97.6 %), | 0.84 mm; |
| 22: 146.2 g | (97.5 %), | 0.83 mm; |
| 23: 147 g | (98 %), | 0.85 mm; |
| 24: 134.3 g | (97 %), | 0.83 mm; |
| 25: 129.3 g | (97 %), | 0.85 mm. |

Table IV

| (Examples 8 and 19 to 25) | | | | | | | | |
|---|---|---|---|---|---|---|---|---|
| Polymer | | Expander | | Degree of Swelling [%] | | Loading Capacity [%] | Release Parameters | |
| Example | Composition | Type and Percentage added | | (1) | (2) | | T-50 (3) | T-90 (4) |
| 8 | 6535 | - | 0 | 32 | 64 | 30 | 2.33 | 21.3 |
| 19 | 6535 | PM-1000 | 5 | 32 | 74 | 30 | 2.7 | 18 |
| 20 | 6535 | PM-1000 | 10 | 29 | 75 | 33.6 | 3.2 | 21 |
| 21 | 6535 | PM-1000 | 15 | 30 | 80 | 35.7 | 3.0 | >21 |
| 22 | 6535 | PM-1000 | 20 | 29 | 82.5 | 36.2 | 3.1 | >21 |
| 23 | 6535 | PM-1000 | 25 | 29 | 85 | 38.4 | 2.7 | >21 |
| 24 | 6535 | PM-1000 | 30 | 28.5 | 88 | 38.0 | 2.2 | 20 |
| 25 | 6535 | PM-1000 | 35 | 30 | 97 | 39.8 | 1.7 | 20 |

(1) Swelling in 100 % water
(2) Swelling in 100 % ethanol
(3) Hours elapsed while releasing 50 % active ingredient
(4) Hours elapsed while releasing 90 % active ingredient

Example 26: Preparation of Copolymer Hydrogel 9010

Using the method of copolymerization described in example 7, the composition of the monomer/macromer is adjusted to 1800 g HEMA dissolving 200 g of isocyanate endcapped polymeg 2000 intermediate. The resulting monomer/macromer is of lower viscosity, accordingly the polymerization is done at 150 rpm stirring rate resulting in a bead product of 0.55 mm average particle size.

Example 27:

Using the process of example 26, the monomer/macromer phase is composed of 144 g monomer/macromer and 36 g of polymeg 2000 as expanding additive. The average bead size of the dry product after alcoholic extraction is 0.98 mm in diameter.

Example 28:

The procedure of example 27 is carried out using polymeg 1000 instead of the polymeg 2000 as the expander-additive. The average bead diameter is 0.86 mm in diameter.

Example 29:

The procedure of example 27 is carried out using polymeg 650 as the expander instead of the polymeg 2000 used above. The average polymer bead size is 0.72 mm in diameter. Below are shown the swelling characteristics of 9010 copolymer hydrogels with diverse molecular weight expander additives (+25 % each). An intermediate ethanol/water medium (55 % ethanol) has been added to the tests.

Table V

| (Examples 26 to 29) | | | |
|---|---|---|---|
| Example | Degree of Swelling [%] in | | |
| | water 100 % | water/ethanol (45:55) | ethanol 100 % |
| 26 | 54 | 152 | 80 |
| 27 | 55 | 226 | 120 |
| 28 | 56 | 195 | 100 |
| 29 | 56 | 175 | 89 |

Example 30:

This example shows the preparation of controlled swelling hydrogels by the use of both hydrophobic co-monomers in the amphiphilic composition and expander additives. Four copolymers are prepared according to the procedure shown in example 8.

a) The unexpanded comparative, unchanged example 8 hydrogel;

b) The unexpanded copolymer containing a hydrophobic co-monomer in the composition: 156.5 g monomer/macromer plus 23.5 g of butyl methacrylate (monomer/macromer b);

c) The a) copolymer expanded as in example 12;

d) The b) copolymer expanded by mixing 51.4 g ethylene glycol monobutyl ether acetate expander to 128.6 g of the monomer/macromer used in the b) run. The products are tested for their swelling behavior:

| | Degree of Swelling [%] in | | |
|---|---|---|---|
| | water 100 % | ethanol 100 % | water/ethanol (25:75) |
| a) | 32 | 64 | |
| b) | 29 | 100 | 137 |
| c) | 45 | 114 | |
| d) | 29 | 148 | 202 |

Example 31:

A variant of sample 30 d) is prepared starting with a 6040 monomer/macromer as described in example 7. 107.2 g thereof are mixed with 21.4 g butyl methacrylate and 51.4 g Ektasolve EBAC. Polymerization is performed as shown in examples 9 to 10.

Comparison of swelling data of the example 31 hydrogel, the example 10 polymer (a 6040 composition with 60 % ethylene glycol monobutyl ether acetate), and those of example 7, without modifying additives, shows the following results:

| Example | Degree of Swelling [%] in | |
|---|---|---|
| | 100 % water | 100 % ethanol |
| 7 | 27 | 60 |
| 10 | 41 | 122 |
| 31 | 23 | 139 |

Example 32:

300 g isophorone diisocyanate/poly-(tetramethylene oxide) condensate macromer of example 1 is dissolved in 1200 g hydroxyethyl methacrylate, containing 1.2 g t-butyl peroctoate. After degassing for one

hour at 66 Pa pressure, 750 g polymeg 2000 are dissolved in the mix. The composition is distributed by a turbine mixer in a 10 liter reactor in the 85 ° C ready aqueous phase of 3,484 g deionized water/868.4 g NaCl/397.2 g of a 50 % $MgCl_2 \cdot 6H_2O$ aqueous solution, which is heated under nitrogen purging to 70 ° C and 934.5 ml 1.0 N NaOH stirred in at 125 rpm with further heating.

Stirring is adjusted to 80 rpm and the system kept at 95-100 ° C for two hours. After thorough washing, the bead polymer is filtered and extracted with hot alcohol until all expanding polymeg 2000 is removed. Vacuum drying at 80-90 ° C and screening completes the preparation.

Example 33:

The procedure of example 32 is carried out with the addition of 50 % poly-(propylene oxide) glycol expander in lieu of polymeg 2000.

Example 34:

Hydrogel beads of both examples 32 and 33 are loaded with carbamazepine in identical runs: 58.3 g of 80:20 dichloromethane/methanol are stirred and 16.7 g carbamazepine are dissolved at room temperature. 20 g Hydrogel beads of a particle size between 40 and 45 mesh are added and stirred for 48 hours. After complete absorption, the material is dried at room temperature and 400 Pa vacuum. The loaded beads in gelatin capsule #0 (USP standard size) containe 200 mg active ingredient. The following release rates are obtained:

| Example | Release Parameters | |
|---|---|---|
| | T-50 (1) | T-90 (2) |
| 32 | 0.4 | 1.9 |
| 33 | 0.5 | 2.25 |

(1) Hours elapsed while releasing 50 % active ingredient
(2) Hours elapsed while releasing 90 % active ingredient

as per USP Apparatus II (100 rpm). Comparative release rate test using 200 mg carbamazepine tablets give T-50 and T-90 values of 0.5 and 3 hours, respectively.

Example 35: Loading of hydrogel with diclofenac sodium

A hydrogel polymer of example 7, in bead form, is soaked in a 75 % by weight solution of diclofenac sodium dissolved in a medium of 88 % methanol and 12 % distilled water. The mixture is rotated at 30-60 revolutions per minute at 39-41 ° C for about 24 hours whereby the solution is inbibed into the beads and equilibrium is achieved. The solvent is then removed by freeze drying at <27 Pa pressure for 3 hours, with additional vacuum drying for 22 hours at room temperature, whereby the loaded hydrogel is achieved.

In the same manner, the hydrogels of example 8, 10, and 19-25 are also loaded with diclofenac sodium.

Example 36:

200 g of the expanded hydrogel beads of example 19 are added to a solution of 132.9 g of diclofenac sodium in a mixture of 139.8 g methanol and 200 g distilled water. The temperature is maintained at about 40 ° C and the mixture rotated for 24 hours. The solvent is removed by freeze drying at 27 Pa over a period of about 3 hours with additional drying at room temperature for about 22 hours to result in a 98 % yield of 30 % diclofenac sodium loaded hydrogel product.

**Claims**

**Claims for the following Contracting States : BE, CH, DE, FR, GB, IT, LI, LU, NL, SE**

**1.** A process for the preparation of a controlled, sustained release composition comprising:
(a) an organic-solvent soluble active agent in an amount sufficient for the total desired dosage during the release period and distributed within
(b) a water-insoluble macromer expanded hydrogel comprising the crosslinked copolymerization

product of

(A) 30 to 90 % by weight of said hydrogel of (a') a water-soluble monoolefinic monomer, or mixture of said monomers, or (b') a water-soluble monoolefinic monomer, or mixture of said monomers with 1 to 50 % by weight of total monomers of a water-insoluble monoolefinic monomer, or mixture of said water-insoluble monomers, with

(B) 70 to 10 % by weight of said hydrogel of a terminal diolefinic hydrophobic macromer having a molecular weight from 400 to 8000, said macromer having the formula I,

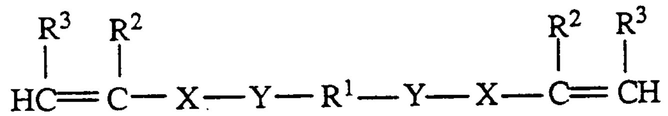

(I)

wherein $R^1$ is a polycondensate chain having a molecular weight from 200 to 4000, which is the residue of a poly (propylene oxide) or poly (tetramethylene oxide) glycol having ether linkages; $R^2$ is hydrogen, methyl or $-CH_2COOR^4$ wherein $R^4$ is hydrogen or an alkyl group with up to ten carbon atoms; $R^3$ is hydrogen or $-COOR^4$, with the proviso that at least one of $R^2$ and $R^3$ is hydrogen; X is oxygen, -COO-, or $-CONR^5-$ wherein $R^5$ is hydrogen or alkyl of up to 5 carbon atoms and Y is a direct bond or the radical $-R^6-Z^1-CO-NH-R^7-NH-CO-Z^2-$, wherein $R^6$ is linked to X and represents branched or linear alkylene of up to 7 carbon atoms, $Z^1$ and $Z^2$ are oxygen or $NR^5$, and $R^7$ is the diradical of an aliphatic, including cycloaliphatic, or aromatic diisocyanate, with the proviso that in case X is oxygen, Y is different from a direct bond, and $R^2$ and $R^3$ are hydrogen; comprising the steps of copolymerizing said monomer (A) and said macromer (B) in the presence of a free radical initiator at a reaction temperature between 20 to 150 °C, in the presence or absence of an inert diluent, to form said hydrogel, and loading said active agent (a) into said hydrogel, characterized in that the copolymerizing step is made in the presence of 5 to 70 weight percent of the total weight of (A) and (B) of a macromer (B) soluble compound of the formula II,

$$R' \left( R'' - O \right)_n R''' \qquad \text{(II)}$$

wherein R' is hydroxy, alkoxy of up to eight carbon atoms or alkanoyloxy of up to eight carbon atoms; R'' is straight or branched chain alkylene of two to six carbon atoms; R''' is hydrogen, alkyl of up to eighteen carbon atoms or alkanoyl of up to eighteen carbon atoms; and n is an integer from 1 to 100, with the proviso that if n is 1 or R'' is ethylene, R''' is alkyl of two to eighteen carbon atoms or alkanoyl of three to eighteeen carbon atoms, or a mixture thereof.

**2.** A process according to claim 1, wherein said active agent is incorporated into said hydrogel by adding said active agent to the monomer (A) and said macromer (B) prior to, or during, said copolymerizing of (A) and (B).

**3.** A process according to claim 1, wherein said active agent is incorporated into said hydrogel by diffusion from an organic solvent medium containing said active agent subsequent to said copolymerizing of (A) and (B).

**4.** A process according to claim 1 or 3, wherein said active agent is a pharmaceutically, insecticidally, or herbicidally effective agent.

**5.** A process according to claim 4, wherein said active agent is a pharmaceutically effective agent.

**6.** A process according to claim 1, wherein R' is hydroxy or alkanoyloxy of up to three carbon atoms, R'' is alkylene of two to four carbon atoms, n is 1 and R''' is alkyl of three to six carbon atoms.

**7.** A process according to claim 1, wherein the compound of formula II is ethylene glycol monobutyl ether.

**8.** A process according to claim 1, wherein the compound of formula II is of the formula III,

$$HO\text{—}(R''\text{—}O)_n\text{—}H \qquad (III)$$

where n has an average value betweeen 8 and 80 and R'' is alkylene of three to four carbon atoms.

**9.** A process according to claim 8, wherein n has an average value between 25 and 80 and R'' is isopropylene.

**10.** A process according to claim 8, wherein n has an average value between 8 and 40 and R'' is tetramethylene.

**11.** A process according to claim 1, wherein said macromer is a polytetramethylene oxide glycol with a molecular weight of 600 to 4000, endcapped with isophorone diisocyanate and reacted with two moles, per mole of said glycol, of a hydroxyalkyl acrylate or methacrylate, wherein alkyl has 2 to 4 carbon atoms.

**12.** A process according to claim 1 or 11, wherein said water-soluble monoolefinic monomer is 2-hydroxyethyl methacrylate.

**13.** A composition produceable according to claim 1.

**14.** A composition produceable according to claim 3.

**15.** A composition produceable according to claim 4.

**16.** A composition produceable according to claim 5.

**17.** A composition produceable according to claim 7.

**18.** A composition produceable according to claim 9.

**19.** A composition produceable according to claim 10.

**20.** A composition according to claim 15, wherein the active agent is diclofenac sodium.

**21.** A composition according to claim 15, wherein the active agent is carbamazepine.

**22.** A composition according to claim 16 for use in a method of orally administering to a patient in need of the same an effective amount of a pharmaceutically effective active agent.

**23.** A composition according to claim 16 for use in a method of orally administering to a patient in need of the same an effective amount of a pharmaceutically effective active agent, wherein said pharmaceutically effective agent is diclofenac sodium.

**24.** A composition according to claim 16 for use in a method of orally administering to a patient in need of the same an effective amount of a pharmaceutically effective active agent, wherein said pharmaceutically effective agent is carbamazepine.

**Claims for the following Contracting State : AT**

**1.** A process for the preparation of a controlled, sustained release composition comprising:
(a) an organic-solvent soluble active agent in an amount sufficient for the total desired dosage during the release period and distributed within
(b) a water-insoluble macromer expanded hydrogel comprising the crosslinked copolymerization

product of

(A) 30 to 90 % by weight of said hydrogel of (a') a water-soluble monoolefinic monomer, or mixture of said monomers, or (b') a water-soluble monoolefinic monomer, or mixture of said monomers with 1 to 50 % by weight of total monomers of a water-insoluble monoolefinic monomer, or mixture of said water-insoluble monomers, with

(B) 70 to 10 % by weight of said hydrogel of a terminal diolefinic hydrophobic macromer having a molecular weight from 400 to 8000, said macromer having the formula I,

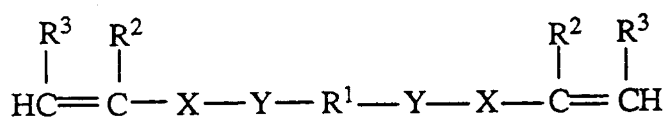

(I)

wherein $R^1$ is a polycondensate chain having a molecular weight from 200 to 4000, which is the residue of a poly (propylene oxide) or poly (tetramethylene oxide) glycol having ether linkages; $R^2$ is hydrogen, methyl or $-CH_2COOR^4$ wherein $R^4$ is hydrogen or an alkyl group with up to ten carbon atoms; $R^3$ is hydrogen or $-COOR^4$, with the proviso that at least one of $R^2$ and $R^3$ is hydrogen; X is oxygen, -COO-, or $-CONR^5-$ wherein $R^5$ is hydrogen or alkyl of up to 5 carbon atoms and Y is a direct bond or the radical $-R^6-Z^1-CO-NH-R^7-NH-CO-Z^2-$, wherein $R^6$ is linked to X and represents branched or linear alkylene of up to 7 carbon atoms, $Z^1$ and $Z^2$ are oxygen or $NR^5$, and $R^7$ is the diradical of an aliphatic, including cycloaliphatic, or aromatic diisocyanate, with the proviso that in case X is oxygen, Y is different from a direct bond, and $R^2$ and $R^3$ are hydrogen; comprising the steps of copolymerizing said monomer (A) and said macromer (B) in the presence of a free radical initiator at a reaction temperature between 20 to 150 °C, in the presence or absence of an inert diluent, to form said hydrogel, and loading said active agent (a) into said hydrogel, characterized in that the copolymerizing step is made in the presence of 5 to 70 weight percent of the total weight of (A) and (B) of a macromer (B) soluble compound of the formula II,

$$R'-(R''-O)_n-R''' \qquad (II)$$

wherein R' is hydroxy, alkoxy of up to eight carbon atoms or alkanoyloxy of up to eight carbon atoms; R'' is straight or branched chain alkylene of two to six carbon atoms; R''' is hydrogen, alkyl of up to eighteen carbon atoms or alkanoyl of up to eighteen carbon atoms; and n is an integer from 1 to 100, with the proviso that if n is 1 or R'' is ethylene, R''' is alkyl of two to eighteen carbon atoms or alkanoyl of three to eighteeen carbon atoms, or a mixture thereof.

2. A process according to claim 1, wherein said active agent is incorporated into said hydrogel by adding said active agent to the monomer (A) and said macromer (B) prior to, or during, said copolymerizing of (A) and (B).

3. A process according to claim 1, wherein said active agent is incorporated into said hydrogel by diffusion from an organic solvent medium containing said active agent subsequent to said copolymerizing of (A) and (B).

4. A process according to claim 1 or 3, wherein said active agent is a pharmaceutically, insecticidally, or herbicidally effective agent.

5. A process according to claim 4, wherein said active agent is a pharmaceutically effective agent.

6. A process according to claim 1, wherein R' is hydroxy or alkanoyloxy of up to three carbon atoms, R'' is alkylene of two to four carbon atoms, n is 1 and R''' is alkyl of three to six carbon atoms.

7. A process according to claim 1, wherein the compound of formula II is ethylene glycol monobutyl ether.

**8.** A process according to claim 1, wherein the compound of formula II is of the formula III,

$$HO-(-R''-O-)_n-H \qquad (III)$$

where n has an average value betweeen 8 and 80 and R'' is alkylene of three to four carbon atoms.

**9.** A process according to claim 8, wherein n has an average value between 25 and 80 and R'' is isopropylene.

**10.** A process according to claim 8, wherein n has an average value between 8 and 40 and R'' is tetramethylene.

**11.** A process according to claim 1, wherein said macromer is a polytetramethylene oxide glycol with a molecular weight of 600 to 4000, endcapped with isophorone diisocyanate and reacted with two moles, per mole of said glycol, of a hydroxyalkyl acrylate or methacrylate, wherein alkyl has 2 to 4 carbon atoms.

**12.** A process according to claim 1 or 11, wherein said water-soluble monoolefinic monomer is 2-hydroxyethyl methacrylate.

**13.** A process according to claim 4, wherein the active agent is diclofenac sodium.

**14.** A process according to claim 4, wherein the active agent is carbamazepine.

**Revendications**
**Revendications pour les Etats contractants suivants : BE, CH, DE, FR, GB, IT, LI, LU, NL, SE**

**1.** Procédé de préparation d'une composition à libération contrôlée et prolongée comprenant :
(a) un agent actif soluble dans un solvant organique en une quantité suffisante pour la posologie totale désirée pendant la période de libération et distribué dans
(b) un hydrogel dilaté avec un macromère insoluble dans l'eau comprenant le produit de copolymérisation réticulé de
(A) 30 à 90% en poids dudit hydrogel de (a') un monomère monooléfinique soluble dans l'eau ou un mélange de tels monomères, ou (b') un monomère monooléfinique soluble dans l'eau, ou un mélange desdits monomères avec de 1 à 50% en poids du total de monomères d'un monomère monooléfinique insoluble dans l'eau, ou d'un mélange desdits monomères insolubles dans l'eau avec
(B) de 70 à 10% en poids dudit hydrogel d'un macromère hydrophobe dioléfinique terminal ayant un poids moléculaire compris entre 400 et 8000, ledit macromère répondant à la formule I,

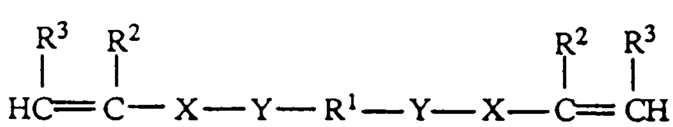
(I)

dans laquelle $R^1$ est une chaîne de produit de polycondensation ayant un poids moléculaire compris entre 200 et 4000, qui est le résidu d'un poly(oxyde de propylène) ou poly(oxyde de tétraméthylène)-glycol ayant des liaisons éthers ; $R^2$ est un hydrogène, un méthyle ou $-CH_2COOR^4$, où $R^4$ est un hydrogène ou un groupe alcoyle ayant jusqu'à 10 atomes de carbone ; $R^3$ est un hydrogène ou $-COOR^4$, sous réserve qu'au moins un des radicaux $R^2$ et $R^3$ est un hydrogène ; X est un hydrogène, -COO-, ou $-CONR^5-$ où $R^5$ est un hydrogène ou un alcoyle ayant jusqu'à 5 atomes de carbone et Y est une liaison directe ou le radical

$-R^6-Z^1-CO-NH-R^7-NH-CO-Z^2-$,

où $R^6$ est lié à X et représente un alcoylène ramifié ou linéaire ayant jusqu'à 7 atomes de carbone, $Z^1$ et $Z^2$ représentent un oxygène ou $NR^5$ et $R^7$ est le diradical d'un diisocyanate aliphatique, y compris cycloaliphatique ou aromatique, sous réserve que dans le cas où X est un oxygène, Y est différent d'une liaison directe, et $R^2$ et $R^3$ représentent un hydrogène ; comprenant les étapes de copolymérisation dudit monomère (A) et dudit macromère (B) en présence d'un inducteur de radicaux libres à une température réactionnelle comprise entre 20 et 150 ° C, en présence ou en l'absence d'un diluant inerte, pour former ledit hydrogel, et d'un chargement dudit agent actif (a) dans ledit hydrogel, caractérisé en ce que l'étape de copolymérisation est conduite en présence de 5 à 70% en poids du poids total de (A) et (B) d'un composé de formule II soluble dans le macromère (B),

$$R'\!-\!\left(R''\!-\!O\right)_n\!R''' \qquad (II)$$

où R' est un hydroxy, alcoxy ayant jusqu'à 8 atomes de carbone ou alcanoyloxy ayant jusqu'à 8 atomes de carbone ; R'' est un alcoylène à chaîne droite ou ramifiée ayant de 2 à 6 atomes de carbone ; R''' est un hydrogène, un alcoyle ayant jusqu'à 18 atomes de carbone ou un alcanoyle ayant jusqu'à 18 atomes de carbone ; et n est un nombre entier allant de 1 à 100, sous réserve que si n vaut 1 ou R'' est un éthylène, R''' est un alcoyle ayant de 2 à 18 atomes de carbone ou un alcanoyle ayant de 3 à 18 atomes de carbone, ou un de leurs mélanges.

**2.** Procédé selon la revendication 1, dans lequel ledit agent actif est incorporé dans ledit hydrogel par addition dudit agent actif au monomère (A) et audit macromère (B) avant ou pendant ladite copolymérisation de (A) et (B).

**3.** Procédé selon la revendication 1, dans lequel ledit agent actif est incorporé dans ledit hydrogel par diffusion à partir d'un milieu solvant organique contenant ledit agent actif après ladite copolymérisation de (A) et (B).

**4.** Procédé selon la revendication 1 ou 3, dans lequel ledit agent actif est un agent à action pharmaceutique, insecticide ou herbicide.

**5.** Procédé selon la revendication 4, dans lequel ledit agent actif est un agent pharmaceutiquement efficace.

**6.** Procédé selon la revendication 1, dans lequel R' est un hydroxy ou un alcanoyloxy ayant jusqu'à 3 atomes de carbone, R'' est un alcoylène ayant de 2 à 4 atomes de carbone, n vaut 1 et R''' est un alcoyle ayant de 3 à 6 atomes de carbone.

**7.** Procédé selon la revendication 1, dans lequel le composé de formule II est le monobutyléther d'éthylène glycol.

**8.** Procédé selon la revendication 1, dans lequel le composé de formule II est de formule III

$$HO\!-\!\left(R''\!-\!O\right)_n\!H \qquad (III)$$

dans laquelle n a une valeur moyenne comprise entre 8 et 80 et R'' est un alcoylène de 3 à 4 atomes de carbone.

**9.** Procédé selon la revendication 8, dans lequel n a une valeur moyenne comprise entre 25 et 80 et R'' est un isopropylène.

**10.** Procédé selon la revendication 8, dans lequel n a une valeur moyenne comprise entre 8 et 40 et R'' est un tétraméthylène.

**11.** Procédé selon la revendication 1, dans lequel ledit macromère est un poly-(oxyde de tétraméthylène)-glycol ayant un poids moléculaire de 600 à 4000, recouvert à son extrémité de diisocyanate d'isophorone et que l'on fait réagir avec 2 moles, par mole dudit glycol, d'un acrylate ou méthacrylate d'hydroxyalcoyle, où l'alcoyle a de 2 à 4 atomes de carbone.

**12.** Procédé selon la revendication 1 ou 11, dans lequel ledit monomère monooléfinique soluble dans l'eau est le méthacrylate de 2-hydroxyéthyle.

**13.** Composition que l'on peut préparer selon la revendication 1.

**14.** Composition que l'on peut préparer selon la revendication 3.

**15.** Composition que l'on peut préparer selon la revendication 4.

**16.** Composition que l'on peut préparer selon la revendication 5.

**17.** Composition que l'on peut préparer selon la revendication 7.

**18.** Composition que l'on peut préparer selon la revendication 9.

**19.** Composition que l'on peut préparer selon la revendication 10.

**20.** Composition selon la revendication 15, dans laquelle l'agent actif est le diclofénac sodique.

**21.** Composition selon la revendication 15, dans laquelle l'agent actif est la carbamazépine.

**22.** Composition selon la revendication 16 pour application dans un procédé d'administration par voie orale à un malade qui en a besoin d'une quantité efficace d'un agent actif pharmaceutiquement efficace.

**23.** Composition selon la revendication 16 pour application dans un procédé d'administration par voie orale à un malade qui en a besoin d'une quantité efficace d'un agent actif pharmaceutiquement efficace, où ledit agent pharmaceutiquement efficace est le diclofénac sodique.

**24.** Composition selon la revendication 16 pour application dans un procédé d'administration par voie orale à un malade qui en a besoin d'une quantité efficace d'un agent actif pharmaceutiquement efficace, où ledit agent pharmaceutiquement efficace est la carbamazépine.

**Revendications pour l'Etat contractant suivant : AT**

**1.** Procédé de préparation d'une composition à libération contrôlée et prolongée comprenant :

(a) un agent actif soluble dans un solvant organique en une quantité suffisante pour la posologie totale désirée pendant la période de libération et distribué dans

(b) un hydrogel dilaté avec un macromère insoluble dans l'eau comprenant le produit de copolymérisation réticulé de

(A) 30 à 90% en poids dudit hydrogel de (a') un monomère monooléfinique soluble dans l'eau ou un mélange de tels monomères, ou (b') un monomère monooléfinique soluble dans l'eau, ou un mélange desdits monomères avec de 1 à 50% en poids du total de monomères d'un monomère monooléfinique insoluble dans l'eau, ou d'un mélange desdits monomères insolubles dans l'eau avec

(B) de 70 à 10% en poids dudit hydrogel d'un macromère hydrophobe dioléfinique terminal ayant un poids moléculaire compris entre 400 et 8000, ledit macromère répondant à la formule I,

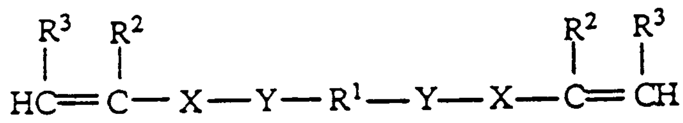

(I)

dans laquelle $R^1$ est une chaîne de produit de polycondensation ayant un poids moléculaire compris entre 200 et 4000, qui est le résidu d'un poly(oxyde de propylène) ou poly(oxyde de tétraméthylène)-glycol ayant des liaisons éthers ; $R^2$ est un hydrogène, un méthyle ou $-CH_2COOR^4$, où $R^4$ est un hydrogène ou un groupe alcoyle ayant jusqu'à 10 atomes de carbone ; $R^3$ est un hydrogène ou $-COOR^4$, sous réserve qu'au moins un des radicaux $R^2$ et $R^3$ est un hydrogène ; X est un hydrogène, -COO-, ou $-CONR^5-$ ou $R^5$ est un hydrogène ou un alcoyle ayant jusqu'à 5 atomes de carbone et Y est une liaison directe ou le radical

$R^6-Z^1-CO-NH-R^7-NH-CO-Z^2-$,

où $R^6$ est lié à X et représente un alcoylène ramifié ou linéaire ayant jusqu'à 7 atomes de carbone, $Z^1$ et $Z^2$ représentent un oxygène ou $NR^5$ et $R^7$ est le diradical d'un diisocyanate aliphatique, y compris cycloaliphatique ou aromatique, sous réserve que dans le cas où X est un oxygène, Y est différent d'une liaison directe, et $R^2$ et $R^3$ représentent un hydrogène ; comprenant les étapes de copolymérisation dudit monomère (A) et dudit macromère (a) en présence d'un inducteur de radicaux libres à une température réactionnelle comprise entre 20 et 150 ° C, en présence ou en l'absence d'un diluant inerte, pour former ledit hydrogel, et d'un chargement dudit agent actif (a) dans ledit hydrogel, caractérisé en ce que l'étape de copolymérisation est conduite en présence de 5 à 70% en poids du poids total de (A) et (a) d'un composé de formule II soluble dans le macromère (B),

$$R'\text{—}(R''\text{—}O)_n\text{—}R''' \qquad \text{(II)}$$

où R' est un hydroxy, alcoxy ayant jusqu'à 8 atomes de carbone ou alcanoyloxy ayant jusqu'à 8 atomes de carbone ; R'' est un alcoylène à chaîne droite ou ramifiée ayant de 2 à 6 atomes de carbone ; R''' est un hydrogène, un alcoyle ayant jusqu'à 18 atomes de carbone ou un alcanoyle ayant jusqu'à 18 atomes de carbone ; et n est un nombre entier allant de 1 à 100, sous réserve que si n vaut 1 ou R'' est un éthylène, R''' est un alcoyle ayant de 2 à 18 atomes de carbone ou un alcanoyle ayant de 3 à 18 atomes de carbone, ou un de leurs mélanges.

2. Procédé selon la revendication 1, dans lequel ledit agent actif est incorporé dans ledit hydrogel par addition dudit agent actif au monomère (A) et audit macromère (B) avant ou pendant ladite copolymérisation de (A) et (B).

3. Procédé selon la revendication 1, dans lequel ledit agent actif est incorporé dans ledit hydrogel par diffusion à partir d'un milieu solvant organique contenant ledit agent actif après ladite copolymérisation de (A) et (B).

4. Procédé selon la revendication 1 ou 3, dans lequel ledit agent actif est un agent à action pharmaceutique, insecticide ou herbicide.

5. Procédé selon la revendication 4, dans lequel ledit agent actif est un agent pharmaceutiquement efficace.

6. Procédé selon la revendication 1, dans lequel R' est un hydroxy ou un alcanoyloxy ayant jusqu'à 3 atomes de carbone, R'' est un alcoylène ayant de 2 à 4 atomes de carbone, n vaut 1 et R''' est un alcoyle ayant de 3 à 6 atomes de carbone.

7. Procédé selon la revendication 1, dans lequel le composé de formule II est le monobutyléther d'éthylène glycol.

**8.** Procédé selon la revendication 1, dans lequel le composé de formule II est de formule III

$$HO\!-\!(\!-R''\!-\!O\!-\!)_n\!-\!H \qquad (III)$$

dans laquelle n a une valeur moyenne comprise entre 8 et 80 et R'' est un alcoylène de 3 à 4 atomes de carbone.

**9.** Procédé selon la revendication 8, dans lequel n a une valeur moyenne comprise entre 25 et 80 et R'' est un isopropylène.

**10.** Procédé selon la revendication 8, dans lequel n a une valeur moyenne comprise entre 8 et 40 et R'' est un tétraméthylène.

**11.** Procédé selon la revendication 1, dans lequel ledit macromère est un poly-(oxyde de tétraméthylène)-glycol ayant un poids moléculaire de 600 à 4000, recouvert à son extrémité de diisocyanate d'isophorone et que l'on fait réagir avec 2 moles, par mole dudit glycol, d'un acrylate ou méthacrylate d'hydroxyalcoyle, où l'alcoyle a de 2 à 4 atomes de carbone.

**12.** Procédé selon la revendication 1 ou 11, dans lequel ledit monomère monooléfinique soluble dans l'eau est le méthacrylate de 2-hydroxyéthyle.

**13.** Procédé selon la revendication 4, dans lequel l'agent actif est le diclofénac sodique.

**14.** Procédé selon la revendication 4, dans lequel l'agent actif est la carbamazépine.

**Patentansprüche**
**Patentansprüche für folgende Vertragsstaaten : BE, CH, DE, FR, GB, IT, LI, LU, NL, SE**

**1.** Verfahren zur Herstellung einer Zusammensetzung mit gesteuerter Langzeitfreisetzung, enthaltend:
(a) einen in einem organischen Lösungsmittel löslichen Wirkstoff in einer für die während des Freisetzungszeitraumes erwünschte Gesamtdosis ausreichenden Menge und verteilt in
(b) einen wasserunlöslichen, makromergequellten Hydrogel enthaltend das vernetzte Copolymerisationsprodukt aus
(A) 30-90 Gew.-%, bezogen auf das Hydrogel, (a') eines wasserlöslichen monoolefinischen Monomers oder einer Mischung solcher Monomere oder (b') eines wasserlöslichen monoolefinischen Monomers oder einer Mischung solcher Monomere mit 1 bis 50 Gew.-%, bezogen auf die Gesamtmonomere, eines wasserunlöslichen monoolefinischen Monomers oder einer Mischung solcher wasserunlöslichen Monomere, mit
(B) 70 bis 10 Gew.-%, bezogen auf das Hydrogel, eines endständig diolefinischen, hydrophoben Makromers mit einem Molekulargewicht von 400 bis 8000, wobei das Makromer die Formel I

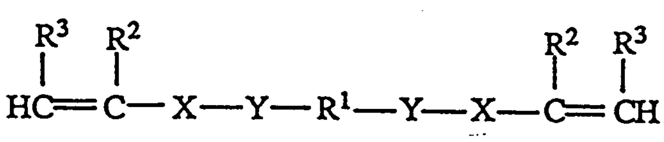

(I)

hat, worin $R^1$ eine Polykondensatkette mit einem Molekulargewicht von 200 bis 4000 ist, die den Rest eines Poly(propylenoxid)- oder Poly(tetramethylenoxid)glykolsmit Etherbindungen darstellt; $R^2$ Wasserstoff, Methyl oder $-CH_2COOR^4$ ist, wobei $R^4$ für Wasserstoff oder eine Alkylgruppe mit bis zu zehn Kohlenstoffatomen steht; $R^3$ Wasserstoff oder $-COOR^4$ ist, unter der Bedingung, daß mindestens eines von $R^2$ und $R^3$ Wasserstoff ist; X Sauerstoff, -COO- oder $-CONR^5$ ist, wobei $R^5$ Wasserstoff oder Alkyl mit bis zu fünf Kohlenstoffatomen ist; und Y eine Direktbindung oder das Radikal-$R^6$-$Z^1$-CO-NH-$R^7$-NH-CO-$Z^2$ ist, worin $R^6$ an X gebunden ist und verzweigtes oder

lineares Alkylen mit bis zu 7 Kohlenstoffatomen darstellt, $Z^1$ und $Z^2$ Sauerstoff oder $NR^5$ sind, und $R^7$ das Diradikal eines aliphatischen, einschließlich cycloaliphatischen, oder aromatischen Diisocyanats ist, unter der Bedingung, daß dann, wenn X Sauerstoff ist, Y keine Direktbindung darstellt und $R^2$ und $R^3$ Wasserstoff sind; umfassend die Schritte des Copolymerisierens des Monomers (A) und des Makromers (B) in Gegenwart eines Radikal-Initiators bei einer Reaktionstemperatur zwischen 20 und 150 °C, in Gegenwart oder Abwesenheit eines inerten Verdünnungsmittels, um das Hydrogel zu bilden, und des Einbringens des Wirkstoffes (a) in das Hydrogel, dadurch gekennzeichnet, daß der Copolymerisierungsschritt in Gegenwart von 5 bis 70 Gew.-%, bezogen auf das Gesamtgewicht von (A) und (B), einer in dem Makromer (B) löslichen Verbindung der Formel II erfolgt,

$$R'\text{-}(\text{-}R''\text{—}O\text{-})_n\text{-}R''' \qquad (II)$$

worin $R^1$ für Hydroxy, Alkoxy mit bis zu acht Kohlenstoffatomen oder Alkanoyloxy mit bis zu acht Kohlenstoffatomen steht; R'' ein gerad- oder verzweigtkettiges Alkylen mit zwei bis sechs Kohlenstoffatomen ist; R''' Wasserstoff, Alkyl mit bis zu achtzehn Kohlenstoffatomen oder Alkanoyl mit bis zu achtzehn Kohlenstoffatomen ist und n eine ganze Zahl von 1 bis 100 ist, unter der Bedingung, daß dann, wenn $n = 1$ oder R'' Ethylen ist, R''' Alkyl mit zwei bis achtzehn Kohlenstoffatomen oder Alkanoyl mit drei bis achtzehn Kohlenstoffatomen oder eine Mischung daraus ist.

2. Verfahren nach Anspruch 1, bei welchem der Wirkstoff in das Hydrogel durch Zusatz des Wirkstoffes zu dem Monomer (A) und dem Makromer (B) vor oder während der Copolymerisation von (A) und (B) eingebracht wird.

3. Verfahren nach Anspruch 1, bei welchem der Wirkstoff in das Hydrogel durch Diffusion aus einem den Wirkstoff enthaltenden organischen Lösungsmittelmedium im Anschluß an die Copolymerisation von (A) und (B) eingebracht wird.

4. Verfahren nach Anspruch 1 oder 3, bei welchem der Wirkstoff ein pharmazeutisch, insektizid oder herbizid wirksames Mittel ist.

5. Verfahren nach Anspruch 4, bei welchem der Wirkstoff ein pharmazeutisch wirksames Mittel ist.

6. Verfahren nach Anspruch 1, bei welchem R' Hydroxy oder Alkanoyloxy mit bis zu drei Kohlenstoffatomen, R'' Algen mit zwei bis vier Kohlenstoffatomen, $n = 1$ und R''' Alkyl mit drei bis sechs Kohlenstoffatomen ist.

7. Verfahren nach Anspruch 1, bei welchem die Verbindung der Formel II Ethylenglykolmonobutylether ist.

8. Verfahren nach Anspruch 1, bei welchem die Verbindung der Formel II der Formel III entspricht

$$HO\text{-}(\text{-}R''\text{—}O\text{-})_n\text{-}H \qquad (III)$$

worin n einen Mittelwert zwischen 8 und 80 besitzt und R'' Alkylen mit drei oder vier Kohlenstoffatomen ist

9. Verfahren nach Anspruch 8, bei welchem n einen Mittelwert zwischen 25 und 80 besitzt und R'' Isopropylen ist.

10. Verfahren nach Anspruch 8, bei welchem n einen Mittelwert zwischen 8 und 40 besitzt und R'' Tetramethylen ist.

**11.** Verfahren nach Anspruch 1, bei welchem das Makromer ein Polytetramethylenoxidglykol mit einem Molekulargewicht von 600 bis 4000, endabgedeckt mit Isophorondiisocyanat und umgesetzt mit zwei Mol pro Mol des Glykols eines Hydroxyalkylacrylats oder -methacrylats, worin das Alkyl zwei bis vier Kohlenstoffatome besitzt, ist.

**12.** Verfahren nach Anspruch 1 oder 11, bei welchem das wasserlösliche monoolefinische Monomer 2-Hydroxyethylmethacrylat ist.

**13.** Zusammensetzung herstellbar nach Anspruch 1.

**14.** Zusammensetzung herstellbar nach Anspruch 3.

**15.** Zusammensetzung herstellbar nach Anspruch 4.

**16.** Zusammensetzung herstellbar nach Anspruch 5.

**17.** Zusammensetzung herstellbar nach Anspruch 7.

**18.** Zusammensetzung herstellbar nach Anspruch 9.

**19.** Zusammensetzung herstellbar nach Anspruch 10.

**20.** Zusammensetzung nach Anspruch 15, bei welcher der Wirkstoff Diclofenac-Natrium ist.

**21.** Zusammensetzung nach Anspruch 15, bei welcher der Wirkstoff Carbamazepin ist.

**22.** Zusammensetzung nach Anspruch 16, für die Verwendung in einem Verfahren zur oralen Verabreichung eines pharmazeutisch wirksamen Wirkstoffes in einer wirksamen Menge an einen Patienten, der eines solchen bedarf.

**23.** Zusammensetzung nach Anspruch 16, für die Verwendung in einem Verfahren zur oraten Verabreichung eines pharmazeutisch wirksamen Wirkstoffes in einer wirksamen Menge an einem Patienten, der eines solchen bedarf, bei welcher das pharmazeutisch wirksame Mittel Diclofenac-Natrium ist.

**24.** Zusammensetzung nach Anspruch 16, für die Verwendung in einem Verfahren zur oralen Verabreichung eines pharmazeutisch wirksamen Wirkstoffes in einer wirksamen Menge an einem Patienten, der eines solchen bedarf, bei welcher das pharmazeutisch wirksame Mittel Carbamazepin ist.

**Patentansprüche für folgenden Vertragsstaat : AT**

**1.** Verfahren zur Herstellung einer Zusammensetzung mit gesteuerter Langzeitfreisetzung, enthaltend:
(a) einen in einem organischen Lösungsmittel löslichen Wirkstoff in einer für die während des Freisetzungszeitraumes erwünschte Gesamtdosis ausreichenden Menge und verteilt in
(b) einen wasserunlöslichen, makromergequellten Hydrogel enthaltend das vernetzte Copolymerisationsprodukt aus
(A) 30-90 Gew.-%, bezogen auf das Hydrogel, (a') eines wasserlöslichen monoolefinischen Monomers oder einer Mischung solcher Monomere oder (b') eines wasserlöslichen monoolefinischen Monomers oder einer Mischung solcher Monomere mit 1 bis 50 Gew.-%, bezogen auf die Gesamtmonomere, eines wasserunlöslichen monoolefinischen Monomers oder einer Mischung solcher wasserunlöslichen Monomere, mit
(B) 70 bis 10 Gew.-%, bezogen auf das Hydrogel, eines endständig diolefinischen, hydrophoben Makromers mit einem Molekulargewicht von 400 bis 8000, wobei das Makromer die Formel I

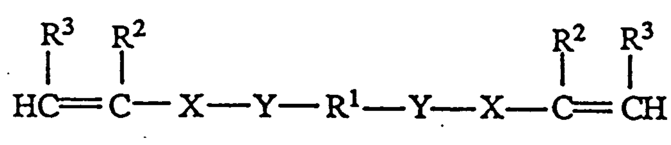

(I)

hat, worin $R^1$ eine Polykondensatkette mit einem Molekulargewicht von 200 bis 4000 ist, die den Rest eines Poly(propylenoxid)- oder Poly(tetramethylenoxid)glykolsmit Etherbindungen darstellt; $R^2$ Wasserstoff, Methyl oder $-CH_2COOR^4$ ist, wobei $R^4$ für Wasserstoff oder eine Alkylgruppe mit bis zu zehn Kohlenstoffatomen steht; $R^3$ Wasserstoff oder $-COOR^4$ ist, unter der Bedingung, daß mindestens eines von $R^2$ und $R^3$ Wasserstoff ist; X Sauerstoff, -COO- oder $-CONR^5$ ist, wobei $R^5$ Wasserstoff oder Alkyl mit bis zu fünf Kohlenstoffatomen ist; und Y eine Direktbindung oder das Radikal-$R^6$-$Z^1$-CO-NH-$R^7$-NH-CO-$Z^2$ ist, worin $R^6$ an X gebunden ist und verzweigtes oder lineares Alkylen mit bis zu 7 Kohlenstoffatomen darstellt, $Z^1$ und $Z^2$ Sauerstoff oder $NR^5$ sind, und $R^7$ das Diradikal eines aliphatischen, einschließlich cycloaliphatischen, oder aromatischen Diisocyanats ist, unter der Bedingung, daß dann, wenn X Sauerstoff ist, Y keine Direktbindung darstellt und $R^2$ und $R^3$ Wasserstoff sind; umfassend die Schritte des Copolymerisierens des Monomers (A) und des Makromers (B) in Gegenwart eines Radikal-Initiators bei einer Reaktionstemperatur zwischen 20 und 150 °C, in Gegenwart oder Abwesenheit eines inerten Verdünnungsmittels, um das Hydrogel zu bilden, und des Einbringens des Wirkstoffes (a) in das Hydrogel, dadurch gekennzeichnet, daß der Copolymerisierungsschritt in Gegenwart von 5 bis 70 Gew.-%, bezogen auf das Gesamtgewicht von (A) und (B), einer in dem Makromer (B) löslichen Verbindung der Formel II erfolgt,

$$R'\text{-}(R''\text{—}O)_n\text{-}R''' \qquad (II)$$

worin R' für Hydroxy, Alkoxy mit bis zu acht Kohlenstoffatomen oder Alkanoyloxy mit bis zu acht Kohlenstoffatomen steht; R'' ein gerad- oder verzweigtkettiges Alkylen mit zwei bis sechs Kohlenstoffatomen ist; R''' Wasserstoff, Alkyl mit bis zu achtzehn Kohlenstoffatomen oder Alkanoyl mit bis zu achtzehn Kohlenstoffatomen ist; und n eine ganze Zahl von 1 bis 100 ist, unter der Bedingung, daß dann, wenn n = 1 oder R'' Ethylen ist, R''' Alkyl mit zwei bis achtzehn Kohlenstoffatomen oder Alkanoyl mit drei bis achtzehn Kohlenstoffatomen oder eine Mischung daraus ist.

**2.** Verfahren nach Anspruch 1, bei welchem der Wirkstoff in das Hydrogel durch Zusatz des Wirkstoffes zu dem Monomer (A) und dem Makromer (B) vor oder während der Copolymerisation von (A) und (B) eingebracht wird.

**3.** Verfahren nach Anspruch 1, bei welchem der Wirkstoff in das Hydrogel durch Diffusion aus einem den Wirkstoff enthaltenden organischen Lösungsmittelmedium im Anschluß an die Copolymerisation von (A) und (B) eingebracht wird.

**4.** Verfahren nach Anspruch 1 oder 3, bei welchem der Wirkstoff ein pharmazeutisch, insektizid oder herbizid wirksames Mittel ist.

**5.** Verfahren nach Anspruch 4, bei welchem der Wirkstoff ein pharmazeutisch wirksames Mittel ist.

**6.** Verfahren nach Anspruch 1, bei welchem R' Hydroxy oder Alkanoyloxy mit bis zu drei Kohlenstoffatomen, R'' Alkylen mit zwei bis vier Kohlenstoffatomen, n = 1 und R''' Alkyl mit drei bis sechs Kohlenstoffatomen ist.

**7.** Verfahren nach Anspruch 1, bei welchem die Verbindung der Formel II Ethylenglykolmonobutylether ist.

**8.** Verfahren nach Anspruch 1, bei welchem die Verbindung der Formel II der Formel III entspricht

$$HO\text{-}(R''\text{—}O)_n\text{-}H \qquad (III)$$

worin n einen Mittelwert zwischen 8 und 80 besitzt und R'' Alkylen mit drei oder vier Kohlenstoffatomen ist.

**9.** Verfahren nach Anspruch 8, bei welchem n einen Mittelwert zwischen 25 und 80 besitzt und R'' Isopropylen ist.

**10.** Verfahren nach Anspruch 8, bei welchem n einen Mittelwert zwischen 8 und 40 besitzt und R'' Tetramethylen ist.

**11.** Verfahren nach Anspruch 1, bei welchem das Makromer ein Polytetramethylenoxidglykol mit einem Molekulargewicht von 600 bis 4000, endabgedeckt mit Isophorondiisocyanat und umgesetzt mit zwei Mol pro Mol des Glykols eines Hydroxyalkylacrylats oder -methacrylats, worin das Alkyl zwei bis vier Kohlenstoffatome besitzt, ist.

**12.** Verfahren nach Anspruch 1 oder 11, bei welchem das wasserlösliche monoolefinische Monomer 2-Hydroxyethylmethacrylat ist.

**13.** Verfahren nach Anspruch 4, bei welchem der Wirkstoff Diclofenac-Natrium ist.

**14.** Verfahren nach Anspruch 4, bei welchem der Wirkstoff Carbamazepin ist.